# EUROPEAN PATENT APPLICATION

(11) **EP 2 962 858 A1**
(43) Date of publication of application: **06.01.2016**
(21) Application number: 15172801.1
(22) Date of filing: 11.05.2011
(51) Int. Cl.: B41J 11/00, B41M 7/00

(54) **ANTIBACTERIAL COATING**

(30) Priority: 11.05.2010 GB 201007853
(62) Divisional of application: 11724441.8
(71) Applicant: Touch Guard Ltd, Huddersfield, Yorkshire HD7 5BG (GB)
(72) Inventor: HINTON-LEVER, Andrew, Huddersfield, Yorkshire HD7 5BG (GB); DUGDALE, Julian, Huddersfield, Yorkshire HD7 5BG (GB)
(74) Representative: Gregory, Robert John H.

(57) **Abstract**

A solidifiable liquid composition suitable as a finishing layer comprises an antibacterial additive; and an adhesion agent to facilitate adhesion of the composition to a substrate. The composition may be used to coat polymeric materials and other items.

## Description

Printed matter, for example, documents, bank notes, books and magazines, advertising material and so on is intended to be held and manipulated by hand. However, printed matter is often intended to be handled by more than one person, and in those circumstances, germs and bacteria can be transferred from one person to the next via the printed matter. The transfer of germs and bacteria, or cross-infection, is generally considered to be undesirable as it can be detrimental to health.

Many institutions and workplaces, such as hospitals, banks, schools, shops, offices and restaurants, have cleaning and cross-infection control procedures in place. Such procedures generally require regular and thorough cleaning of equipment, utensils, tools etc. Nevertheless, institutions often house a great number of portable documents which are regularly handled by various people, but the documents themselves are not subject to strict anti-cross-contamination procedures. In many cases the documents cannot be cleaned because to do so may damage them. It is therefore unsurprising that even despite strict hygiene procedures being in place, cross-infection incidents continue to occur on a fairly regular basis.

Various attempts have been made to reduce this risk, which have tended to concentrate on the use of antibacterial impregnated papers and/or inks. These solutions have not met with overwhelming commercial success for economic and efficacy reasons. Specifically, antibacterial impregnated papers may require expensive new tooling in their manufacture and since only the surface of the paper comes into contact with the user, the relatively expensive antibacterial agents within the bulk of the material are largely ineffective. To impregnate the bulk of the paper, when only the surface of it comes into contact with users is therefore uneconomic and wasteful. On the other hand, antibacterial impregnated inks have also been shown to be largely ineffective since only a portion of the paper's surface Is covered therewith, thereby leaving a larger proportion of the paper unprotected.

A need therefore exists for a more effective means of reducing cross-infection as a result of document and packaging handling. An object of this Invention is thus to address, reduce and/or eliminate the risks of cross-infection posed by the manual handling of documents and other printed matter.

According to a first aspect of the invention, there is provided a method of printing comprising a printing step wherein one or more layers of print are deposited onto one or both sides of a substrate; and a finishing step wherein a substantially continuous outer protective layer is applied to the entire surface of one or more sides of the substrate; wherein the outer protective layer comprises an antibacterial additive.

According to an embodiment of the invention, the finishing step comprises applying a finishing composition to a transfer surface; and transferring the finishing composition from the transfer surface to the substrate to form the continuous outer protective later; the finishing composition comprising the antibacterial additive.

According to an embodiment of the invention, the transfer surface is the surface of a roller.

According to an embodiment of the invention, the printing step is performed on a first area of the substrate at the same time as the finishing step is performed on a second area of the substrate.

The method is preferably carried out using printing techniques, e.g. lithography, thereby making it cost-effective to implement.

According to an embodiment of the invention, the printing method is any one or more of the group comprising a lithographic process, an analox process, a Heidelberg process, a web offset process and a Tresu process.

According to a second aspect of the invention, there is provided printed matter produced by a method of printing comprising a printing step wherein one or more layers of print are deposited onto one or both sides of a substrate; and a finishing step wherein a substantially continuous outer protective layer is applied to the entire surface of one or more sides of the substrate; wherein the outer protective layer comprises an antibacterial additive.

The substrate may be any paper or indeed any printable substrate, including, but without limitation to, paper, card, cardboard and sheet polymers.

The antibacterial additive preferably contains silver ions, and the concentration of silver ions in the outer protective layer is preferably sufficient to impart antibacterial activity thereto.

The invention therefore differs from the antibacterial impregnated papers and inks inasmuch as the antibacterial protection is derived from the use of a continuous outer layer. The use of a continuous outer layer also means that the entire surface of the printed matter (i.e. the un-printed regions between the print, and the print itself) is overlaid with an antibacterial layer. It therefore becomes difficult, if not impossible, to touch any unprotected part of the printed matter, thereby significantly reducing the risk of cross-infection.

Silver ion technology is believed to be particularly suited to this application because of its long-lasting antibacterial activity, Its longevity and durability.

In a preferred embodiment of the invention, the outer protective layer comprises a varnish comprising silver ions. By only doping the outer layer with silver ions, the materials costs can be significantly reduced, compared to the cost of impregnating the bulk of the paper.

According to a third aspect of the invention, there is provided an apparatus comprising a printing stage wherein one or more layers of print are deposited onto one or more sides of a substrate; and a finishing stage wherein a substantially continuous outer protective layer is applied to the entire surface of one or more sides of the substrate.

According to an embodiment of the invention, the finishing stage comprises a transfer surface for receiving a finishing composition and for transferring the finishing composition to the substrate.

According to an embodiment of the invention, the transfer surface Is a roller.

According to an embodiment of the invention, the finishing composition comprises an antibacterial additive.

According to a third aspect of the invention, there is provided a solidifiable liquid composition suitable as a finishing layer for printed matter comprising an antibacterial additive comprising silver ions; and an adhesion agent to facilitate adhesion of the composition to a substrate.

The composition may comprise an aqueous or organic solution of silver ions, or preferably a hydrosol/organosol suspension of silver ion particles.

According to an embodiment of the invention, the adhesion agent comprises any one or more of the group comprising a natural resin, a synthetic resin, rosin, a polymer, a monomer, a binder, a film former, an adhesive, a top coat, a varnish, acrylic, modified oil, linseed oil, polyurethane, protein resin, shellac, acrylate and epoxy.

According to a further embodiment, the composition comprises a solvent.

According to a further embodiment the solvent comprises any one or more of the group comprising water, alcohol, an ester, vegetable oil and a petroleum distillate.

According to a further embodiment the composition further comprises any one or more additives from the group comprising a metal, a matting agent, a wax, a gloss agent, a surface modifier, a pigment, a dye, a dryer, a cross linker, an accelerator and an anti-slip agent.

According to a further embodiment, the composition comprises a 0.1% concentration of silver additive powder in an oil-based coating.

According to a further embodiment, the composition comprises a 1.0% concentration of silver additive liquid in a water-based coating.

According to an embodiment, the antibacterial activity meets or exceeds the requirements of JIS Z2801/ISO22196:2007 for MRSA and E.coli.

A preferred embodiment of the invention shall now be described, by way of example only, with reference to the accompanying drawings in which:
Figure 1 is a schematic diagram showing a manufacturing process for printed matter in accordance with the invention.
Figure 2 is a schematic diagram showing another manufacturing process for printed matter in accordance with the invention.

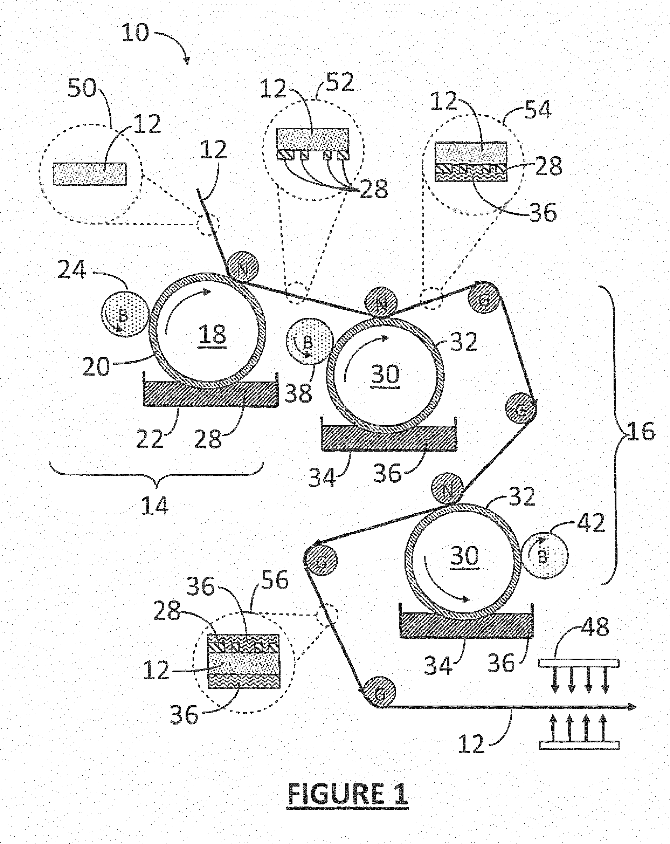

In Figure 1, a lithographic printing press 10 comprises a series of rollers arranged to guide and print onto a continuous web of paper 12. The paper web 12 generally travels from top left to bottom right in the diagram, contacting various rollers in turn. Rollers designated N are nip rollers, those designated G are guide rollers and those designated B are blanket rollers. The printing process comprises two main stages, namely a printing stage 14 and a finishing stage 16.

The printing portion 14 of the press 10 comprises a cylindrical print roller 18, which is arranged to rotate about its longitudinal axis. A printing plate 20, comprising non-printing (negative), hydrophilic (lipophobic) areas and printing (positive), hydrophobic (lipophilic) areas, is wrapped around, and bonded to, the cylindrical print roller 18.

During rotation of the print roller 18, the surface of the printing plate 20 passes through a bath 22 of liquid printing composition 28. The liquid printing composition 28 comprises oil-based ink and a water phase and selectively coats the printing plate 20: the oil-based printing ink 28 adhering to the positive printing areas of the printing plate; and the negative, non-printing areas of the printing plate being protected from ink 28 by the water phase.

As the print roller 18 rotates, it contacts a first blanket roller 24 which squeezes off water on the non-printing, negative area of the printing plate 20. This step prevents the water from wetting the paper 12.

A web of plain, unprinted paper 12, as shown in cross-section in inset 50, is fed into the press 10 from a roll (not shown) of stock and passes between the surface of the print roller 18 and a nip roller N. In this step, an impression of the ink 28 on the print roller 18 is thus transferred onto one side of the paper web 12, as shown in inset 52. The paper web 12 then progresses through the press 10 into the finishing stage 16.

The finishing stage 16 operates in a similar manner to the printing stage 14. A takeup roller 30 having a hydrophobic surface 32, which passes through a bath 34 of oil-based finishing composition 36. The finishing composition 36 comprises an oil-based carrier and a suspension 0.1% suspension of silver ions, which coats the entire surface 30 of the take up roller 30. Excess finishing composition 36 is removed by a second blanket roller 38.

Meanwhile, the web of paper 12 is directed between the take up roller 30 and a second nip roller 40 thereby transferring a layer the finishing composition 36 over the entire surface of the paper web 12, and overlying the previously-deposited ink 28, as shown in inset 54.

The paper web 12 is then passes through a second finishing stage, which coats the opposite side of the web 12. The second finishing stage also comprises a take up roller 30 having a hydrophobic surface 32, which passes through a bath 34 of oil-based finishing composition 36. Again, the finishing composition 36 comprises an oil-based carrier and a suspension 0.1% suspension of silver ions, which coats the entire surface 30 of the take up roller 30. Excess finishing composition 36 Is removed by a third blanket roller 42. The web of paper 12 is directed between the second take up roller 30 and a further nip roller 46 thereby transferring a layer the finishing composition 36 over the entire surface of the opposite side of the paper web 12, as shown in inset 56.

Finally, the paper web 12 is passed through a dryer 48 to cure/dry the ink and finishing composition. The drying step 48 may comprise the application of any one or a combination of heat and/or infra red radiation and/or ultra violet radiation. Depending on the type of ink used, the drying process initiates and drives reactive curing of the ink, and/or accelerates absorption and drying.

It will be appreciated that the printing process could be adapted to include any number of printing and/or finishing stages, provided that the final outermost layer deposited on the web is continuous and comprises antibacterial additives.

In a preferred embodiment, the finishing composition 36 comprises: a resin or varnish, whose primary function is to facilitate adhesion of the ink to the paper; a solvent whose primary functions are to act as a carrier for the silver ions; and a silver ion-containing additive, which gives the coating its antibacterial properties. Alternatively, the finishing composition could comprise an equivalent water-based suspension of silver ions. The material (silver ions) is added generally in a liquid form and a carrier liquid is used in most cases (but not exclusively) this would be ethanol based dispersion as supplied. This would be either stirred into or by way of recirculation pump incorporated into the varnish finishing material. This is specific to the water based finishes for oil based finishes the silver ion is supplied as a powder form and is either milled or stirred into the varnish dependant on required particle size.

The resin/varnish can be any natural or synthetic resin, rosin, polymer or monomer, or blend thereof, which acts as a binder, film former, adhesive, top coat, varnish or similar, such as acrylic, modified oil, linseed oil, polyurethane, protein resin, shellac, acrylate, epoxy. The carrier solvent can be any compound or mixture of compounds, generally liquid, designed to allow the printing process to occur and then cease to interact with the coating by reacting, evaporating, absorption, dissipating, becoming sterically hindered/encapsulated, or similar. Examples of commonly used solvents are water, alcohols, esters, vegetable oils and petroleum distillates. Additional additives may be provided, to modify the coating's luster, texture etc., e.g. mentals, matting agents, waxes, gloss agents, surface modifiers, pigments, dyes, driers, cross linkers, accelerators and anti-slip agents.

In the present invention, the outer layer comprises an oil-based ink and can be applied using a lithography perfecting process. For example, a Heidelberg perfecting press could be used to print conventional ink on the sheet then to apply the outer protective layer all over the paper. The paper can then be turned over (perfected) and the outer protective layer applied to the entirety of the opposite side of the paper thus ensuring that both sides of the paper are fully coated by the protective outer layer.

In an alternative embodiment of the invention, the protective outer layer can be applied using a web offset process, in which case both sides of the paper are coated simultaneously with the protective outer layer.

In a yet further alternative embodiment of the Invention, a water-based protective outer layer can be applied using a doctor blade and chamber system (i.e. Tresu). In such a situation, the protective outer layer is pumped into interior chamber of an analox roller in liquid form, which liquid fills the cells on the analox cylinder. A doctor blade or (or doctor blades) can be used to skim off any excess coating. The protective coating in the cells can then be transferred to the blanket roller and then transferred onto the printed paper just like in conventional coating.

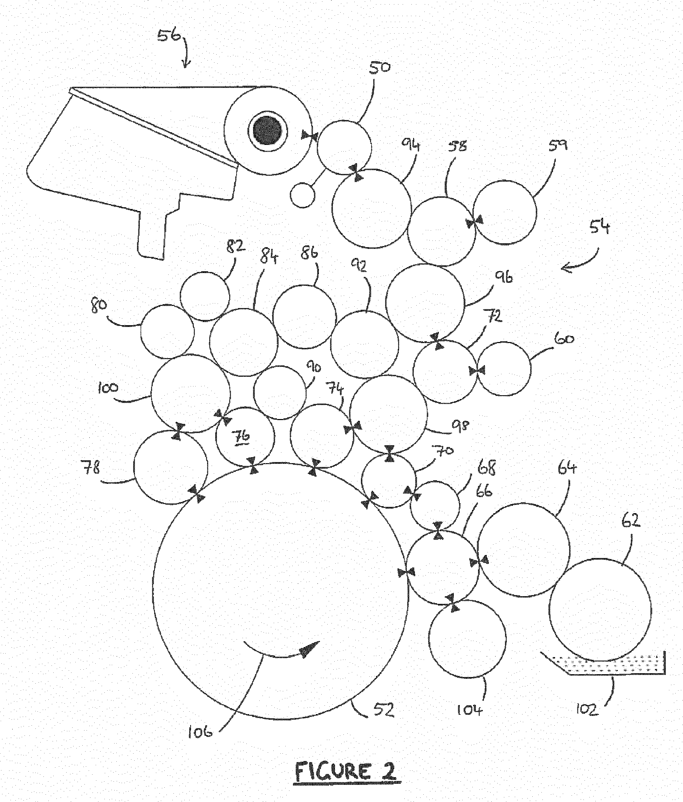

Figure 2 shows part of a standard printing press for printing an image in a single colour onto a substrate. Ink is distributed from roller 50 to roller 52 via a roller train, shown generally at 54. Duct 56 applies ink to roller 50. Rollers 50, 58, 59, 60, 62, 64, 66, 68, 70, 72, 74, 76, 78, 80, 82, 84, 86, 90, 92 are rubber rollers which are removable. Rollers 94, 96, 98, 100 are fixed steel rollers which are not removable, and which oscillate to ensure the even spread of ink.

A printing plate (not shown) is attached to roller 52. The image provided by the printing plate is transferred onto a blanket roller (not shown) and then in turn onto an image roller (not shown). A sheet roller (not shown) then transfers the image from the image roller to a substrate, such as paper (not shown).

A bath 102 contains a dosed water which is used in the printing process to keep images clear. Roller 104 controls damp when ink is being applied to the plate on roller 52.

The direction of rotation of roller 52 is shown by curved arrow 106.

Pairs of small solid triangles in figure 2 indicate contact between adjacent rollers. The stripped setting between two rollers for optimal contact and ink transfer will be known by the person skilled in the art.

The apparatus of Figure 2, as so far described, is a known device for printing an image in a single colour onto a substrate. Additional units as those illustrated in Figure 2 are required for each additional colour used in the printing stage of the printing process. Full colour printing is achieved by using several of the units illustrated in Figure 2, each applying ink of a single colour. According to the present invention, an additional unit as illustrated in Figure 2 is used for applying a finishing composition to one side of a substrate. The finishing composition is applied to the substrate after all the single ink colour images have each been applied by their respective separate individual printing units. The finishing composition is applied in the same manner as applying a coloured ink. The finishing composition is applied through duct 56 onto roller 50, through roller train 54 to a plate (not shown) attached to roller 52. The finishing composition is transferred from the plate (not shown) attached to roller 52 to the substrate (not shown) via, in turn, the blanket roller (not shown), the image roller (not shown) and the sheet roller (not shown).

The finishing composition comprises an antibacterial material and may be composed as described in the previous embodiment.

Figure 2 shows only one printing unit for applying either one of a set of ink colours, or a finishing composition. A perfecting press can incorporate between 2 and 12 of the units as illustrated in Figure 2. A perfecting unit (not shown) is a system situated generally at the centre of a printing press that rotates a substrate for printing on both sides of the substrate in a single pass through the printing press.

The protective outer layer comprises a curable/solidifiable liquid (oil or water based) containing an additive comprising silver ions. The concentration of silver ions is sufficient to render the surface of the paper inert to bacterial growth on the surface. The coating, when dried/cured is inert. Testing to JIS Z2801/ISO22196:2007 for MRSA and E.coli have shown that the protective outer layer protects to the required levels under the above ISO requirements.

ISO 22196:2007, formerly JIS Z 2801:2000, which is a standard relating to antibacterial activity specifies a method of evaluating the antibacterial activity of antibacterial-treated plastic products and other non-porous materials including paint films. According to the standard, a value for the antibacterial activity is determined although there Is no pass/fail criterion. Testing to ISO 22196:2007 involves applying a bacterial suspension to a test specimen and to control specimens, which are then incubated for 24 hours.

Bacteria on the test and control samples are counted before and after incubation, and using a formula provided in the standard, the log of the difference between the before and after counts Is determined to give a measurement of antimicrobial activity. This can also be expressed as percentage kill.

ISO 22196:2007 specifies the use of two bacterial species, namely Staphylococcus aureus and Escherichia coll, however, testing is routinely carried out using other bacteria as well, e.g. MRSA, Pseudomonas, Listeria, Salmonella, Bacillus, Acinebacter etc., in addition to fungi such as Aspergillus and Candida.

In the present case, six 50mm x 50mm samples were coated with the finishing composition for each bacteria/fungus tested. The antibacterial activity of the finishing composition was tested according to a "non-suspended inoculum (NS1)" method, in which the bacteria are applied to the test specimens as a non-suspended inoculum, which is considered to be a more realistic approach for many coatings/surfaces because the test conditions more closely resemble in-use conditions.

The JIS Z 2801 (Antimicrobial Surface Test) is designed to quantitatively measure the ability of an antimicrobial surface to kill or inhibit the growth of microorganisms. The test microorganism is prepared, usually by growth in a liquid culture medium. The suspension of test microorganism is standardized by dilution in a nutritive broth (this affords microorganisms the potential to grow during the test). Control and test surfaces are inoculated with microorganisms, in triplicate, and then the microbial inoculum is covered with a thin, sterile film. Covering the inoculum spreads it, prevents it from evaporating, and ensures close contact with the antimicrobial surface. Microbial concentrations are determined at "time zero" by elution followed by dilution and plating. A control is also run to verify that the neutralization/elution method effectively neutralizes the antimicrobial agent in the antimicrobia surface being tested. Inoculated, covered control and antimicrobial test surfaces are allowed to incubate undisturbed in a humid environment for 24 hours and after incubation, microbial concentrations are determined. The reduction of microorganisms relative to initial concentrations and the control surface is calculated.

The JIS Z 2801 test is quantitative and results tend to be reproducible, provided the inoculum does not spill off of the target area after being covered with the thin film. The method also tests for both bacteriostatic (growth-inhibiting) and bactericidal (bacteria-killing) properties. Because the microbial concentrations are standardized, and bacteria are provided with nutrients during the incubation period, the bacteria are provided with ample opportunity to grow if surfaces are not sufficiently antimicrobial. This is in contrast to certain other antimicrobial tests, where microbes are "incubated" in non-nutritive suspensions, which itself may be stressful over long periods. The method stipulates triplicate experimentation, which helps researchers estimate the precision of the individual tests and increases overall experimental accuracy and includes a rating system for the calculated levels of antimicrobial activity observed in test samples, making determinations of antimicrobial activity less discretionary.

However, the JIS Z 2801 method is not necessarily representative of actual surface contamination events, since a relatively dilute liquid microbial inoculum is spread over a considerable surface area, and then is kept wet (usually for a period of 24 hours). In most real situations, however, microbial contaminants dry quickly onto surfaces, which limits the time that an aqueous medium is available to facilitate interaction between the antimicrobial surface and microorganisms. This means that JIS Z 2801 is a "best-case" sort of test for many products.

The compositions that have been shown to exhibit the above antibacterial activity are: 0.1% concentration of silver additive powder in the oil-based coating; and 1.0% concentration of silver additive liquid in the water-based coating. Additives that have been used successfully are commercially available grades of "BIOMASTER AT300"™ and "BtOMASTER GC100"™, which contain silver chloride / titanium composite particles (silver chloride in a protective titanium oxide sponge, 1 - 3 microns), and an anionic gel.

Laboratory test results on printed samples according to the invention are shown in table 1 below

**Table 1-Test results for antibacterial activity**

| Specimen | Bacteria | Concentration before | Concentration after 24h | Log₁₀ | % kill |
|---|---|---|---|---|---|
| WATER-BASED COATING + BIOMASTER AT300 | MRSA | 2.2E+04 | 9.2E+02 | 1.4 | 95.82% |
| Control | E coli | 2.1E+04 | 2.9E+03 | | |
| WATER-BASED COATING + BIOMASTER AT300 | E coli | 2.1E+04 | < 11.11 | ≥3.28 | ≥99.95% |
| OIL-BASED COATING + BIOMASTER GC100 | E coli | 2.1E+04 | < 11.11 | ≥ 3.28 | ≥99.95% |
| CONTROL | MRSA | 2.5E+04 | 1.6E+04 | | |
| OIL-BASED COATING + BIOMASTER GC100 | MRSA | 2.5E+04 | <11.11 | ≥3.35 | ≥ 99.96% |
| WATER-BASED COATING + BIOMASTER AT300 | MRSA | 2.2E+04 | 9.2E+02 | 1.4 | 95.82% |

The applications for the coating according to the invention are considerable and include (but not exclusive to) finishes to food packaging, menus, bank notes, hospital paperwork and any paper board finishes that are touched by humans and therefore could transfer by touch infection.

### THE FOLLOWING STATEMENTS ARE NOT CLAIMS BUT DESCRIBE SOME EMBODIMENTS OF THE INVENTION

1. A method of printing comprising
   a printing step wherein one or more layers of print are deposited onto one or both sides of a substrate; and
   a finishing step wherein a substantially continuous outer protective layer is applied to the entire surface of one or more sides of the substrate;
   wherein the outer protective layer comprises an antibacterial additive.
2. The method of printing of statement 1 wherein the finishing step comprises
   applying a finishing composition to a transfer surface; and
   transferring the finishing composition from the transfer surface to the substrate to form the continuous outer protective later;
   the finishing composition comprising the antibacterial additive.
3. The method of printing of statement 2 wherein the transfer surface is the surface of a roller.
4. The method of printing of any of statement 1 to statement 3 wherein the printing step is performed on a first area of the substrate at the same time as the finishing step is performed on a second area of the substrate.
5. The method of printing of any of statement 1 to statement 4 wherein the printing method is any one or more of the group comprising a lithographic process, an analox process, a Heidelberg process, a web offset process and a Tresu process.
6. Printed matter produced using the method of any of statement 1 to statement 5.
7. An apparatus comprising
   a printing stage wherein one or more layers of print are deposited onto one or more sides of a substrate; and
   a finishing stage wherein a substantially continuous outer protective layer is applied to the entire surface of one or more sides of the substrate.
8. The apparatus of statement 7 wherein the finishing stage comprises a transfer surface for receiving a finishing composition and for transferring the finishing composition to the substrate.
9. The apparatus of statement 8 wherein the transfer surface is a roller.
10. The apparatus of any of statement 7 to statement 9 wherein the finishing composition comprises an antibacterial additive.
11. A solidifiable liquid composition suitable as a finishing layer for printed matter comprising
   an antibacterial additive comprising silver ions; and
   an adhesion agent to facilitate adhesion of the composition to a substrate.
12. The composition of statement 11 further comprising an aqueous or organic solution of silver ions.
13. The composition of statement 11 or statement 12 further comprising a hydrosol or organosol suspension of silver ion particles.
14. The composition of any of statement 11 to statement 13 wherein the adhesion agent comprises any one or more of the group comprising a natural resin, a synthetic resin, rosin, a polymer, a monomer, a binder, a film former, an adhesive, a top coat, a varnish, acrylic, modified oil, linseed oil, polyurethane, protein resin, shellac, acrylate and epoxy.
15. The composition of any of statement 11 to statement 14, further comprising a solvent.
16. The composition of statement 15 wherein the solvent comprises any one or more of the group comprising water, alcohol, an ester, vegetable oil and a petroleum distillate.
17. The composition of any of statement 11 to statement 16, further comprising any one or more additives from the group comprising a metal, a matting agent, a wax, a gloss agent, a surface modifier, a pigment, a dye, a dryer, a cross linker, an accelerator and an anti-slip agent.
18. The composition of any of statement 11 to statement 17 comprising a 0.1% concentration of silver additive powder in an oil-based coating.
19. The composition of any of statement 11 to statement 17 comprising a 1.0% concentration of silver additive liquid in a water-based coating.
20. A method, printed matter, apparatus or a composition according to any preceding statement, wherein the antibacterial activity meets or exceeds the requirements of JIS Z2801/ISO22196:2007 for MRSA and E.coli.
21. A method, printed matter, apparatus or a composition according to any preceding statement, wherein the substrate comprises any one or more of the group comprising paper, card, cardboard and a polymer.
22. A method, printed matter, an apparatus or a composition as hereinbefore described with reference to, and as illustrated in, the accompanying drawings.

## Claims

1. A solidifiable liquid composition suitable as a finishing layer comprising
an antibacterial additive; and
an adhesion agent to facilitate adhesion of the composition to a substrate.

2. A composition as claimed in claim 1 comprising an aqueous or organic solution of silver ions.

3. A composition as claimed in any of claim 1 or claim 2 comprising a hydrosol or organosol suspension of silver ion particles.

4. The composition as claimed in any of claim 1 to claim 3 wherein the adhesion agent comprises any one or more of the group comprising a natural resin, a synthetic resin, rosin, a polymer, a monomer, a binder, a film former, an adhesive, a top coat, a varnish, acrylic, modified oil, linseed oil, polyurethane, protein resin, shellac, acrylate and epoxy.

5. A composition as claimed in any of claim 1 to claim 4, further comprising a solvent.

6. A composition as claimed in claim 5 wherein the solvent comprises any one or more of the group comprising water, alcohol, an ester, vegetable oil and a petroleum distillate.

7. A composition as claimed in any of claim 1 to claim 6, further comprising any one or more additives from the group comprising a metal, a matting agent, a wax, a gloss agent, a surface modifier, a pigment, a dye, a dryer, a cross linker, an accelerator and an anti-slip agent.

8. A composition as claimed in any of claim 1 to claim 7 comprising a 0.1% concentration of silver additive powder in an oil-based coating.

9. A composition as claimed in any of claim 1 to claim 7 comprising a 1.0% concentration of silver additive liquid in a water-based coating.

10. A method of providing a substrate with antibacterial properties comprising the step of coating said substrate with a composition as defined in any preceding claim.

11. A method as claimed in claim 10 wherein said substrate comprises a polymer.

12. A method of preparing antibacterial food packaging comprising the method of claim 10 or claim 11.

13. A method as claimed in any of claims 10 to 12 further comprising a preceding step of printing onto the substrate.

14. A product obtainable by the method of any of claims 10 to 13.

15. Food packaging obtainable by the method of any of claims 10 to 13, or packaged food comprising said packaging.
